# EUROPEAN PATENT SPECIFICATION

(11) **EP 1 649 886 B1**
(45) Date of publication and mention of the grant of the patent: **02.04.2008**
(21) Application number: 05077995.8
(22) Date of filing: 21.07.2000
(51) Int. Cl.: A61M 15/00, A61J 3/07

(54) **Unit dose capsules for a dry powder inhaler**
Einzelportionskapseln für einen Trockenpulverinhalator
Capsules à dose unitaire pour un inhalateur à poudre sèche

(30) Priority: 23.07.1999 US 145464 P; 22.05.2000 US 206123 P
(43) Date of publication of application: 26.04.2006
(62) Divisional of application: 00960184.0
(73) Proprietor: MannKind Corporation, Valencia, CA 91355 (US)
(72) Inventor: Steiner, Solomon S., Mount Kisco New York 10507 (US); Poole, Trent, South Amherst Massachusetts 01002 (US); Feldstein, Robert, Yonkers New York 10701 (US); Fog, Per B., Bedford Hills New York 10507 (US)
(74) Representative: Powell, Timothy John

(56) References cited:
- EP-A- 0 143 524
- EP-A- 0 180 543
- EP-A- 0 388 621
- EP-A- 0 666 085
- EP-A- 0 844 007
- WO-A-91/19524
- GB-A- 716 815
- GB-A- 2 148 841
- US-A- 3 669 113
- US-A- 4 487 327
- US-A- 5 476 093

## Description

### FIELD OF THE INVENTION

The present invention is in the field of inhalers.

### BACKGROUND OF THE INVENTION

In the early 1970's it was found that certain medicines could be administered in dry-powder form directly to the lungs by inhalation through the mouth or inspiration through the nose. This process allows the medicine to bypass the digestive system, and may, in certain cases, allow smaller does to be used to achieve the same results as orally ingested or injected medicines. In some cases, it provides a delivery technique that reduces side effects for medicines taken by other methods.

Inhaler devices typically deliver their medicinal in a liquid mist or a powder mist. The liquid mist is typically created by a chlorofluorocarbon propellant. However, with the ban on chlorofluorocarbons by the Montreal protocol, interest has turned to dry powder inhalers.

For a dry powder inhaler to work effectively, it must deliver fine particles of medicinal powder that do not agglomerate, and do not end up striking, and being absorbed by the patient's mouth or upper oropharyngeal region. Air flow must therefore not be too fast. Furthermore, it should not be difficult for a patient to load with medicine or to use with the proper technique. Current dry particle inhalers fail in one or more of these important criteria.

EP-A-0 666 085 discloses an inhaler in the form of a mouth tube into which a capsule, containing a powdered medicament, is insertable. The capsule is perforated to allow expulsion of the powder, but the arrangement of EP-A-0 666 085 fails to address the problems identified above.

EP-A-0 388 621 discloses an integrated inhaler and capsule assembly. The capsule is captured within the inhaler during manufacture. EP-A-0 388 621 therefore does not concern a capsule *per se,* and does not seek to provide a discrete design that addresses the problems of the prior art.

### SUMMARY OF THE INVENTION

In its broadest aspect the invention is as defined in Claim 1 hereof. Further, optional features of the invention are as set out in the dependent claims.

Described herein is a dry powder inhaler comprising an intake section; a mixing section, and a mouthpiece. The mouthpiece is connected by a swivel joint to the mixing section, and may swivel back onto the intake section and be enclosed by a cover. The intake chamber comprises a special piston with a tapered piston rod and spring, and one or more bleed-through orifices to modulate the flow of air through the device. The intake chamber further optionally comprises a feedback module to generate a tone indicating to the user when the proper rate of airflow has been achieved. The mixing section holds a capsule with holes containing a dry powder medicament, and the cover only can open when the mouthpiece is at a certain angle to the intake section. The mixing section further opens and closes the capsule when the intake section is at a certain angle to the mouthpiece. The mixing section is a Venturi chamber configured by protrusions or spirals to impart a cyclonic flow to air passing through the mixing chamber. The mouthpiece includes a tongue depressor, and a protrusion to contact the lips of the user to tell the user that the DPI is in the correct position. An optional storage section, with a cover, holds additional capsules. The cover for the mouthpiece, and the cover for the storage section may both be transparent magnifying lenses.

The capsules may be two-part capsules where each portion has apertures which correspond to apertures in the other half when each half is partially fitted to the other half, and fully fitted to the other half. All the apertures may be closed when the two halves are rotated around their longitudinal axes with respect to each other. Each capsule may have a unique key on each half that only fits with a particular inhaler.

Therefore it is an object of the invention to provide a capsule for a dry particle inhaler where particles of drug are dispersed finely.

This and other objects will be readily apparent upon a reading of the present specification, claims and drawings.

### BRIEF DESCRIPTION OF THE SEVERAL VIEWS OF THE DRAWINGS

Figure 1 is a schematic view of the dry particle inhaler described herein.

Figure 2 is schematic view of the mouthpiece cover.

Figure 3 is schematic view showing the angle between the intake section and the mouthpiece.

Figure 4 is a schematic view of the dry particle inhaler, showing the storage section.

Figure 5 is a schematic view of the intake section of the dry particle inhaler, showing the flow regulator and the feedback module.

Figure 6 is a schematic view of the mixing section.

Figure 7 is a schematic view of a capsule to hold medicament.

Figure 8 is a schematic view of the mouthpiece.

Figure 9 is a perspective view of a specific, not claimed, embodiment of the dry particle inhaler in the closed position, with a capsule inserted into the mixing section, and extra capsules stored in the storage section.

Figure 10 is a perspective view of a specific, not claimed, embodiment of the dry particle inhaler showing a capsule being loaded in to the mixing section.

Figure 11 is a perspective view of a specific, not claimed, embodiment of the dry particle inhaler showing a capsule inserted into the mixing section, and the mouthpiece extended for use.

Figures 12, 13, 14, and 15 follow each other in temporal sequence.

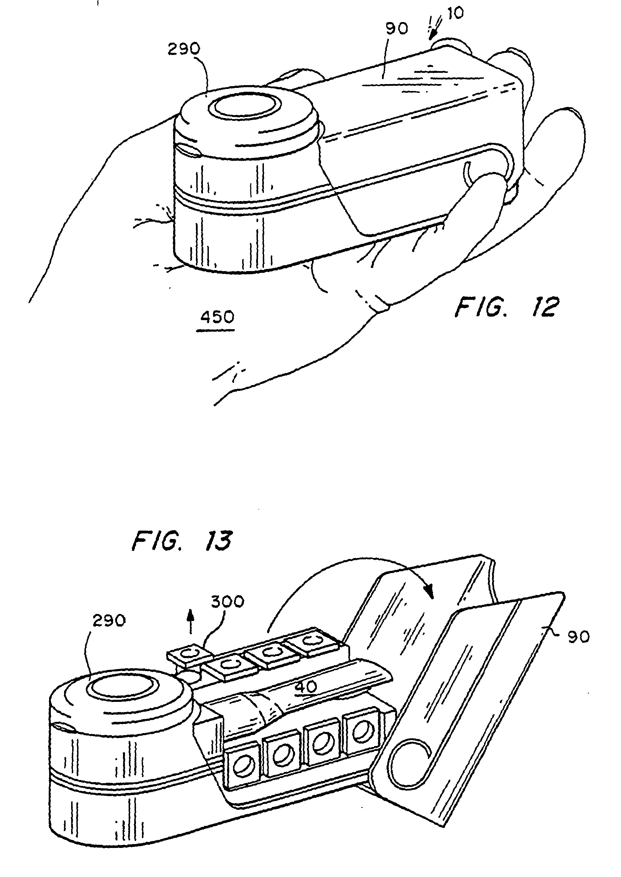

Figure 12 is a perspective view of a specific, not claimed, embodiment of the dry particle inhaler showing a closed mouthpiece cover.

Figure 13 is a perspective view of a specific, not claimed, embodiment of the dry particle inhaler showing an open mouthpiece cover.

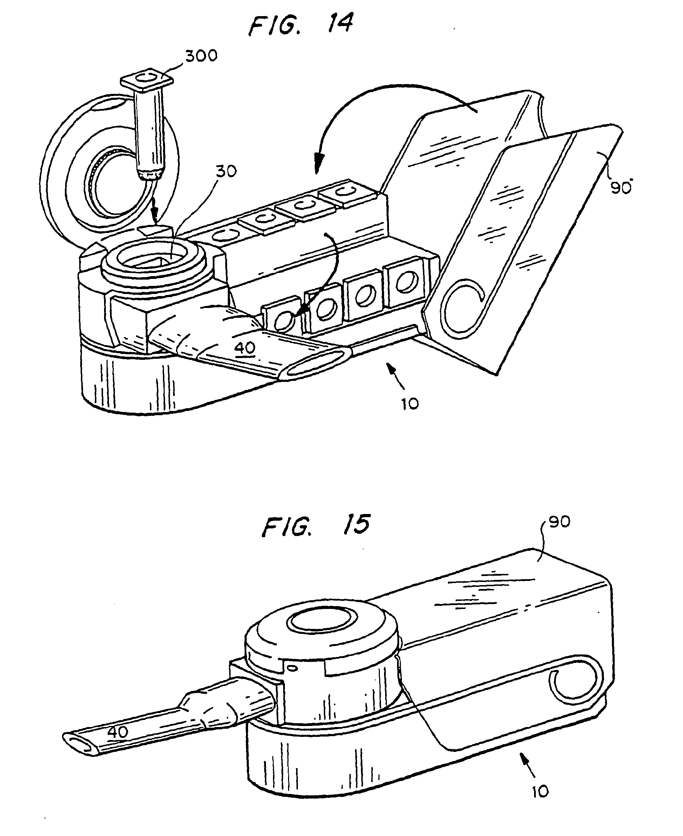

Figure 14 is a perspective view of a specific, not claimed, embodiment of the dry particle inhaler showing an open mouthpiece cover, an open mixing section cover, and a capsule about to be insened into the mixing section.

Figure 15 is a perspective view of a specific, not claimed, embodiment of the dry particle inhaler showing the mouthpiece extended for use.

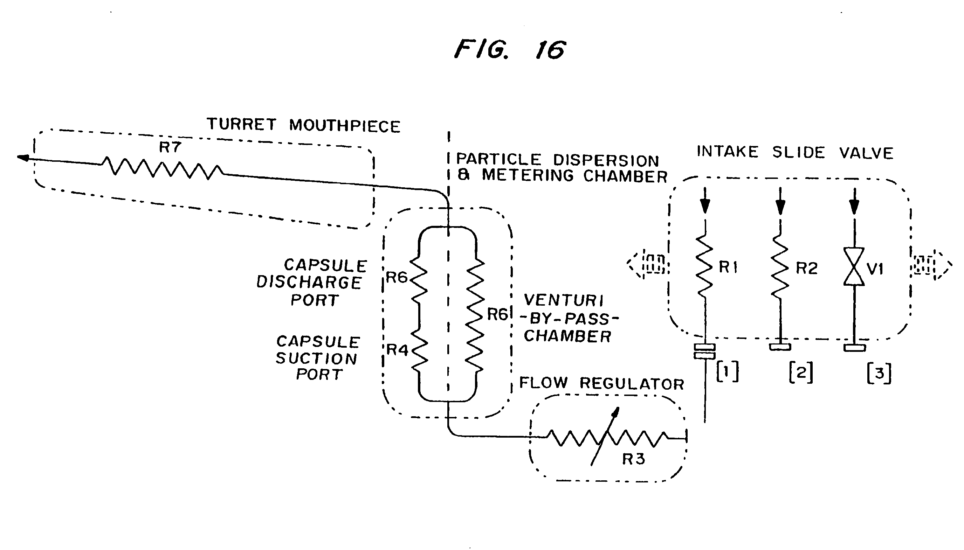

Figure 16 is a view of a pneumatic circuit, where air flows (fluid flows) are represented by their electrical equivalents.

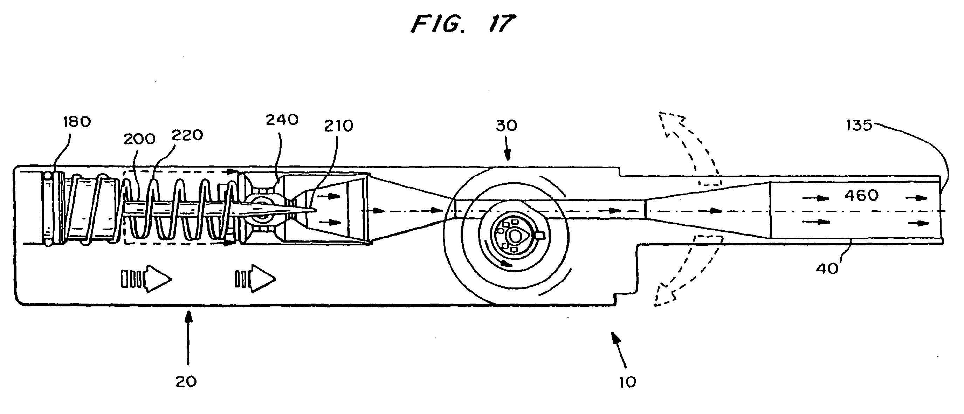

Figure 17 is a schematic view of the dry particle inhaler.

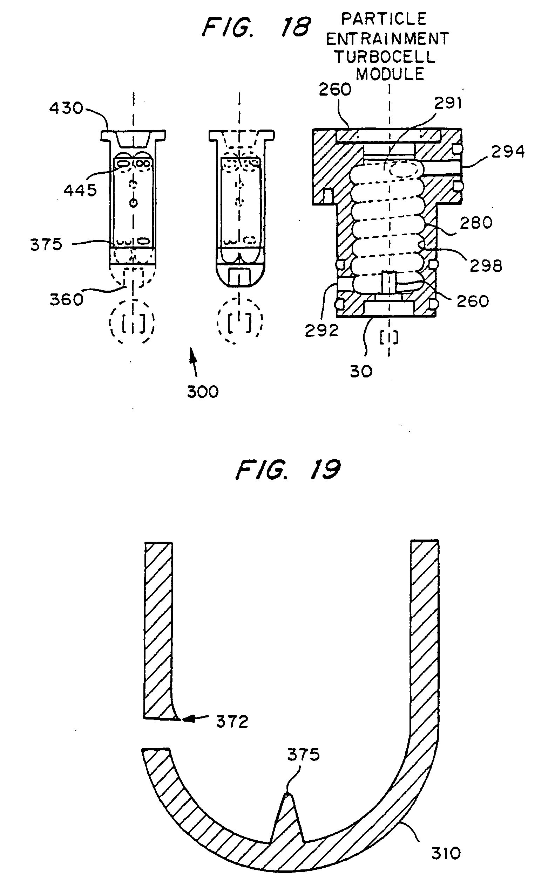

Figure 18 is a cutaway view of a capsule and a portion of the mixing section.

Figure 19 is a cutaway view of half of a capsule, showing a cone in the interior and a secondary hole with a chamfered, or beveled, edge.

### TABLE OF REFERENCE NUMBERS

- 10: dry powder inhaler device
- 20: intake section
- 30: mixing section
- 40: mouthpiece
- 50: air passage through dry powder inhaler device
- 60: longitudinal axis of intake section
- 70: longitudinal axis of mouthpiece section
- 80: swivel joint connecting mouthpiece and mixing section
- 90: cover for mouthpiece
- 100: protrusions on mouthpiece cover
- 110: depressions on dry particle inhaler cover to mate with protrusions on mouthpiece cover
- 120: tongue depressor on mouthpiece
- 130: protrusion on surface of mouthpiece to contact lips of device user
- 135: opening of mouthpiece to be fitted into user's mouth
- 140: intake port
- 150: flow regulator
- 160: bleed orifice
- 170: piston
- 180: piston head
- 190: piston rod
- 200: proximal portion of piston rod
- 210: distal portion of piston rod
- 220: spring
- 230: inner walls of intake section inner chamber
- 240: feedback module
- 250: mechanical fasteners in storage section
- 260: holder in mixing section for capsule
- 270: Venturi chamber
- 280: spiral shape or protrusions to impart cyclonic flow to air
- 290: cover for mixing chamber
- 291: interior of mixing section
- 292: air flow entrance to mixing section
- 294: air flow exit from mixing section
- 296: latch mechanism for mixing section cover
- 298: interior wall of mixing section
- 300: capsule
- 310: first tube
- 320: open end of first tube
- 330: closed end of first tube
- 340: long axis of first tube
- 350: protrusion on first tube
- 360: keying surface on first tube
- 370: secondary holes in first tube
- 372: chamfered edge of secondary hole
- 375: cone in interior of first tube
- 380: second tube
- 390: open end of second tube
- 400: closed end of second tube
- 410: long axis of second tube
- 420: protrusion on second tube
- 430: keying surface on second tube
- 440: secondary holes in second tube
- 445: cone in interior of second tube
- 450: hand of user
- 460: air flow direction
- 470: storage section
- 480: storage section cover

### DETAILED DESCRIPTION OF THE INVENTION

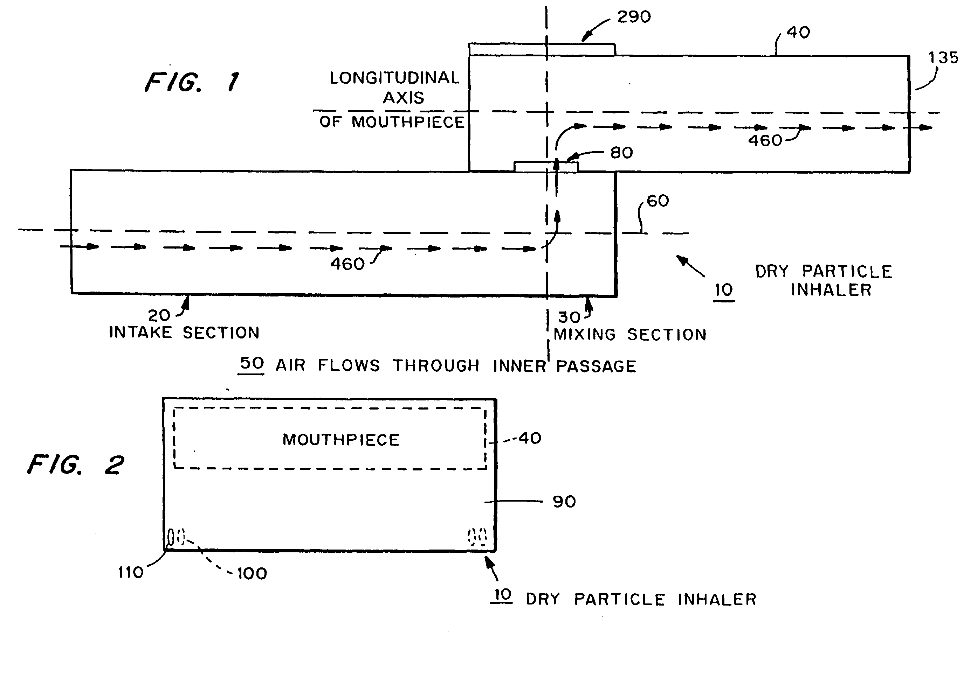

Figure 1 is a schematic drawing of the dry powder inhaler (10) described herein. It comprises an intake section (20), a mixing section (30) and a mouthpiece (40). An air passage (50) goes through the intake section (20), a mixing section (30) and a mouthpiece (40). A swivel joint (80) connects the mouthpiece (40) to the mixing section (30). The mixing section (20) has a cover (290) which may be a transparent magnifying lens. Arrow (460) shows the direction of air flow through the air passage (50) through the dry powder inhaler (10).

Figure 2 shows the mouthpiece cover (90) in the closed position over the dry particle inhaler (10). Protrusions (100) on the mouthpiece cover (90) mate with grooves or depressions (110) on the dry particle inhaler (10), to join the mouthpiece cover (90) to the dry particle inhaler (10).

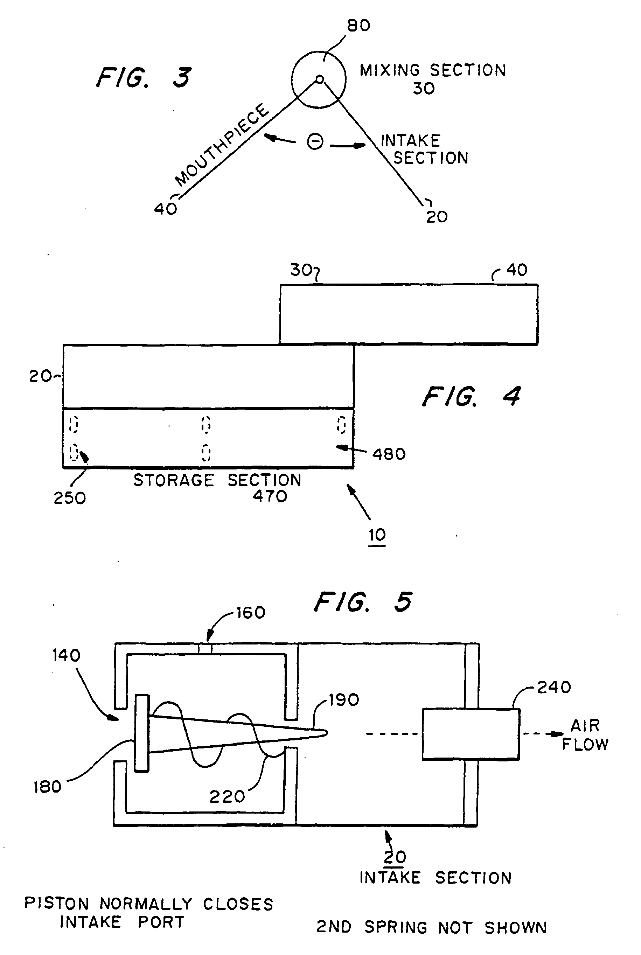

Figure 3 is a schematic of the showing the mouthpiece (40) and the intake section (20) as represented by the longitudinal axis of the mouthpiece (70) and the longitudinal axis of the intake section (60). The swivel joint (80) connecting the mouthpiece (40) to the intake section (20) at the mixing section (30) may be regarded as the vertex of the angle. The importance of the angle (here called theta) between these two longitudinal axes will be further explained.

Figure 4 shows the dry particle inhaler (10) with a storage section (470). Indicated as being inside the storage section (470) are mechanical fasteners (250) which operate to hold medicament capsules (300) (not shown in this Figure) in the storage section. The storage section (470) is shown as appended to the intake section (20). The storage section has a cover (480) which may be a transparent magnifying lens, to allow the user to easily read writing on medicament capsules stored therein. The storage section cover (480) may swivel outward, or slide open on a track (not shown), or open by a variety of mechanisms known to those of skill in the art.

Figure 5 shows the intake section (20) of the dry particle inhaler (10). The direction of air flow is shown by the arrow (460). Air is admitted through an intake port (140) and one or more bleed orifices (160) [The bleed orifices may also be styled as secondary ambient air intake ports]. The piston (170) normally covers the intake port (140). When the user (not shown) inspires, the piston head (180) is drawn backwards, at a steady rate modulated by the spring (220). The spring (220) is fixed to the piston (170) and the inner wall (230) of the intake section chamber. Thus the rate of air flow is controlled. The air flow is further controlled by the tapering of the piston rod (190), past which the air flows. For further control of the air flow, a second spring (not shown) may also control the rate of movement of the piston (170).

The piston (170) and spring (220) combination allow the user (not shown) to generate a vacuum in his lungs before the intake port (140) opens. Thus, by the time enough vacuum is generated to open the intake port (140), there will be sufficient air flow at a sufficient rate in the dry particle inhaler (10) to draw most of the medicament in the capsule (not shown) out of the inhaler into the proper place in the lungs of the user.

A feedback module (240) generates a signal to the user (not shown), which tells the user whether he is inspiring at the correct rate. The signal may be an audible one, in one embodiment a tone that is at a steady pitch when air flow is at a certain steady rate. In one embodiment of the dry particle inhaler (10), which is not claimed, the signal is generated mechanically, such as be a musical reed. In another embodiment which is also not claimed, the signal might be generated electronically, after electronic measurement of the air flow rate. The feedback module (240) would include a means for increasing or lessening the signal strength, or turning the signal off entirely. If the signal were generated by a reed, the mechanism for turning off the signal might be covering a bleed orifice which might admit the air flow generating the signal. If the signal were generated electronically, a simple push button or dial might turn on and off the signal.

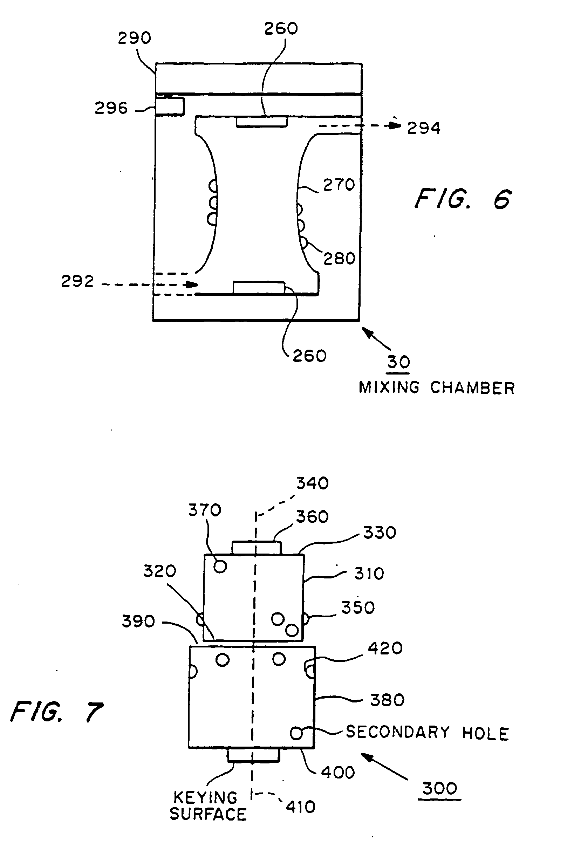

Figure 6 shows a schematic of the mixing section (30). The mixing section has a cover (290), and a holder (260) for a medicament capsule (not shown). The holder (260) is a mechanism which grips and turns the capsule (not shown) to open and close it as the longitudinal axis (70) of the mouthpiece is rotated about the swivel joint (80) relative to the longitudinal axis (60)of the intake section. Such a mechanism may be straightforward: in a simplest embodiment, both the top and bottom halves (not shown) of the capsule could be fixed to their respective holders (260).

The Venturi chamber (270) speeds the flow of air near the capsule (not shown). Air flows in at (292), and out through (294). In one not claimed embodiment, air flows both through and around a capsule (not shown) holding a dry powder medicament. The special shape of the Venturi chamber (270), which further includes protrusions or spiral shapes (280), imparts a cyclonic flow to the air passing through the mixing section (30). This helps to de-agglomerate particles of dry powder. The spiral shape of the interior of the mixing section (291) can be two separate spirals, in one embodiment of the invention. Mixing section (30) therefore provides the means whereby air flow is speeded up to suspend dry particles in air and de-agglomerate them, and then slow the air flow somewhat while the particles are still suspended in air. The cover (290) for the mixing section (30) may be a transparent magnifying lens, so that any writing on the capsule (not shown) may be read easily.

In one not claimed embodiment of the dry particle inhaler (10), the cover (290) of the mixing section may not be opened unless the longitudinal axis (70) of the mouthpiece forms a certain angle with the longitudinal axis (60) of the intake section, with the vertex of the angle being the swivel joint (80) connecting the mouthpiece (40) and the mixing section (30). The latch mechanism (296) for the cover (290) of the mixing section can accomplish this, by any of several mechanical means known to those of ordinary skill in the art. In the simplest embodiment, a catchment (not shown) in the cover (290) for the mixing chamber would be engaged by a slip ring (not shown) on the mixing section which was only a certain number of degrees of a circle. When the mouthpiece (40) were rotated enough relative to the intake section (20), the slip ring (not shown) would no longer engage the catchment (not shown). In one embodiment, the user could open the cover (290) when the angle were between approximately ninety and one-hundred and eighty degrees.

Figure 7 shows a medicament capsule (300) for use with an inhaler, be it a dry powder inhaler (10), or a liquid mist inhaler. The capsule (300) has two halves which fit together, here styled a first tube (310) and a second tube (380). Each tube has an open end (320, 390), and a closed end (330, 400). Each tube also has a long axis (340, 410). In addition, each tube has a number of secondary holes (370, 440). The first tube (310) fits inside the second tube (380) snugly. A protrusion (350) on the outer surface of the first tube (310) can slide past a corresponding protrusion (420) on the inner surface of the second tube (380). This locks the first tube (310) to the second tube (380). Therefore the first tube (310) and the second tube (380) have both an unlocked and a locked position. In the unlocked position, at least one secondary hole (370) in the first tube aligns with at least one secondary hole (440) in the second tube. This permits introduction of a medicament (not shown) into the capsule through the aligned secondary holes (370, 440). The first tube (310) may then be locked to the second tube (380). When a user (not shown) is ready to use a capsule (300), he simply places it in the holder (260) in the mixing section (30), and closes the cover (290). When the holder (260) rotates the first tube (310) around its long axis (340) relative to the second tube (380) and its long axis (410) (the axes are now coincident), that causes at least two secondary holes (370) in the first tube to align with at least two secondary holes (440) in the second tube. Air can now pass in, through, and out of the capsule (300), releasing the medicament contained therein. In one embodiment of the inhaler, which is not claimed the capsule (300) might open when the angle between the longitudinal axis (70) of the mouthpiece section, the vertex of the swivel joint (80), and the longitudinal axis (70) of the mouthpiece section were between one hundred and seventy and one-hundred and eighty degrees. This rotation of the mouthpiece (40) relative to the intake section (20) would cause a corresponding rotation of the first tube (310) about its long axis (340) relative to the second tube (380) and its long axis (410).

In one embodiment of the invention, several protrusions on the surfaces of the first tube or the second tube might provide a variety of locking positions. Similarly, a variety of secondary holes in the first and second tubes might provide a variety of rotational positions aligning or not aligning secondary holes on the first and second tubes.

The capsules described herein permit the introduction of liquid or gel medicament which can be dried in the capsule, creating a powder. This permits the accurate production of very small amounts of powdered medicament in a capsule, since it can be formed from a larger volume of accurately metered liquid or gel medicament. This permits very accurate microdosing. In addition, chemical reactions and drug mixtures may be made directly in the capsules described herein, then the resulting formulation dried.

In one embodiment of the capsule (300), one or more of the secondary holes (370, 440) used to admit air to the capsule is oval-shaped (elliptical). In one embodiment of the invention, the ratio of the long axis of the ellipse to the shorter axis may be between 1:1 and 3:1, and may be 2:1. This ratio may be called a vertical aspect ratio. In one embodiment of the invention, the intersection of the surface defining one or more of the secondary holes (370, 440) and the surface defining the interior of the capsule (300) meet in a chamfered, or beveled, edge. This chamfered edge creates a vortex when air flows through the secondary holes (370, 440).

Each capsule (300) also has a keying surface (or fastening mechanism) on the closed end (330) of the first tube and the closed end (400) of the second tube comprising the capsule. The keying surface (360) on the first tube may be different from the keying surface (430) on the second tube. That permits easy tactile and visual identification of the orientation of the capsule. It also permits a system where each drug formulation in a capsule (300) corresponds to a dry particle inhaler (10), so users cannot mix up drugs. In one embodiment of the invention, the keying surface (360) of the first tube mates with a keying surface (430) of a different second tube, or the mechanical fasteners (250) of the storage section (470). This permits easy storage of the capsules (300) in the storage section (470).

Figure 18 shows a medicament capsule (300), with a keying surface (360) on the first tube and a keying surface (430) on the second tube. It also shows a cutaway view of the mixing section (30) and the air flow entrance (292) to the mixing section and the air flow exit (294) to the mixing section. A spiral shape (280) is given to the interior walls (298) of the mixing section, to impart a cyclonic flow to air passing through. The air flow entrance (292) and air flow exit (294) in this embodiment are tangential to the imaginary tube we might call the mixing section interior (291). That is to say, if a radius were drawn perpendicular to the long axis of the tube, and a tangent line were drawn to the circle perpendicular to the radius, the air flow would exit the mixing section along that tangent line. The tangential air flow exit (294) increases the velocity of the air flow, and thus helps disperse the medicament particles. As can be seen from Figure 18, the mixing section interior (291) is sized to accommodate a medicament capsule (300). Keying mechanisms (360, 430) are shaped to mate with holder (260) in the mixing section. Capsules according to the present invention may have a number of shapes, including ovoid and rectangular shapes. A variety of shapes of protrusions and slots may also be employed as keying surfaces. For instance, a keying surface might be a rectangular block, and a capsule holder might have a rectangular orifice. Alternatively, a keying surface might be triangular, hexagonal, Z-shaped, C-shaped, etc., and the holder would have the correspondingly shaped aperture.

Figure 18 also shows one embodiment of the capsule (300) where a cone (375) is located in the interior of the first tube, and a cone (445) is located in the interior of the second tube. These cones (375, 445) cause the air flow within the capsule to be cyclonic, aiding in mixing the medicament particles with the air. A cone is shown herein, but other cyclone-creating structures are contemplated by the present invention.

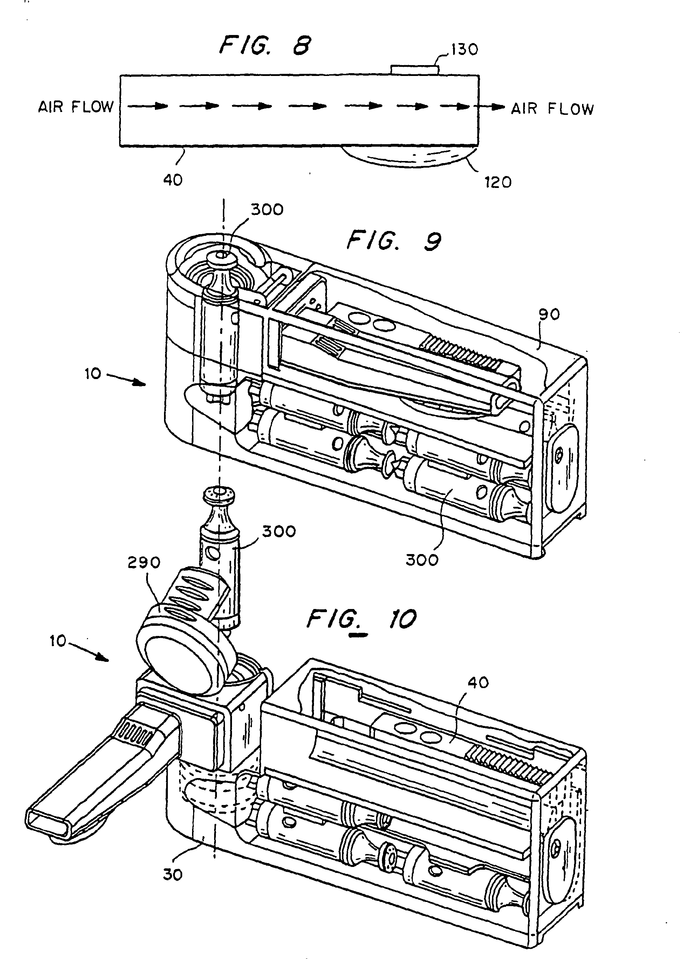

Figure 8 shows the mouthpiece (40) of the dry particle inhaler (10). It has a protrusion (130) on its surface to contact the lips of a user (not shown). This helps the user place the mouthpiece correctly in his mouth. The mouthpiece (40) also includes a tongue depressor (120), which may have a bulbous shape. The mouthpiece (40) is long enough that it fits approximately midway into the user's mouth (not shown). This permits greater delivery of medicament to the lungs, and less delivery to the oral cavity. The mouthpiece (40) has a particular aspect ratio of its inner channel (50) (see Figure 17). This slows the air passing through the channel so that the air borne particulates do not end up striking the back of the user's throat. However, the air is not slowed so much that the particulates settle out of the air flow.

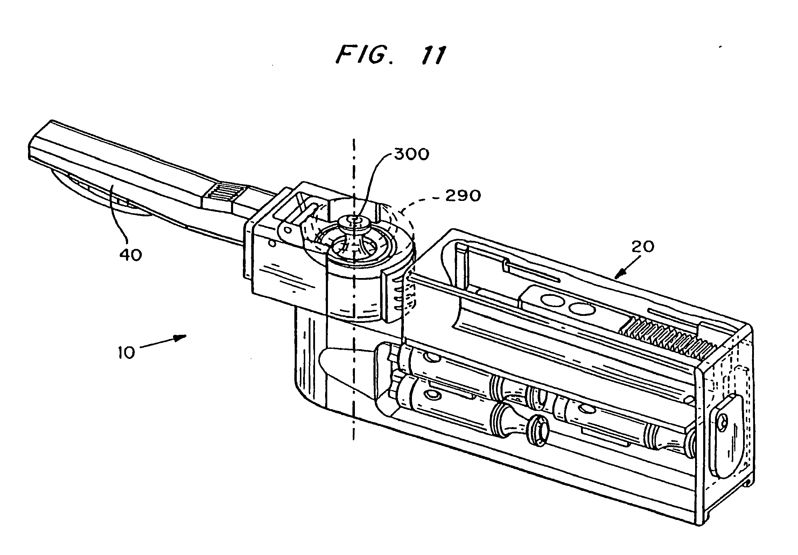

Figure 9, Figure 10, and Figure 11 show one specific embodiment of the dry particle inhaler (10) which is not claimed. In Figure 9, the cover (90) of the mouthpiece is closed, and several capsule (300) are in the storage section (470). In Figure 10, the mouthpiece (40) has been rotated relative to the intake section (20). The longitudinal axis (60) [not shown] of the intake section here makes an approximately ninety degree angle with the longitudinal axis (70) of the mouthpiece section. This permits the cover (290) for the mixing section to be opened. A medicament capsule (300) taken from the storage section (470) is about to be inserted into the mixing section (30). In Figure 11, the mouthpiece (40) has been rotated to a fully extended position, the cover (290) for the mixing section has been closed, and the dry particle inhaler 910) is ready for use. In one embodiment of the dry particle inhaler (10), when the dry particle inhaler is in the closed position (Figure 9), the interior of the intake section (20) would be isolated from the outside air, but the mouthpiece (40) interior and the mixing section interior (291) would not be, permitting them to dry out after being exposed to the humid breath of a user.

Figure 12, Figure 13, Figure 14, and Figure 15 show a temporal sequence where a capsule (300) of medicament is loaded into the mixing section (30) of a dry particle inhaler (10), and the mouthpiece (40) is extended for use. The dry particle inhaler (10) described herein can also be used for nasal delivery of medicaments. A small tube (not shown) can be fitted to the end of the mouthpiece (40), and the other end of the tube inserted into the nostril. Alternatively, the mouthpiece (40) may be replaced by a nosepiece (not shown), whose free end is sized to be inserted into a nostril of a user. In another not claimed embodiment, a device such as a bellows or a syringe is used to force air through the dry particle inhaler (10) into a nosepiece inserted into the nostril of a user (not shown).

Figure 16 shows the fluid (air) flow of the dry particle inhaler (10) modeled as the equivalent electrical circuit. This is styled a "pneumatic resistance circuit".

Figure 17 shows a schematic view of the dry particle inhaler (10). The air passage (50) through the dry particle inhaler widens as it goes through the mouthpiece (40) along the direction of the air flow (460). The opening (135) of the mouthpiece to be inserted into the mouth of the user may be roughly ellipsoid, or oval, and thus have a major axis and a minor axis. The ratio of these two may be called the horizontal aspect ratio. In one not claimed embodiment of the inhaler, the horizontal aspect ratio is between 2:1 and 4:1. In one not claimed embodiment of the dry particle inhaler (10), the horizontal aspect ratio is 3:1. Shaping the opening (135) in this manner keeps the drug particles collimated, maintains the optimal velocity of the particles in the air stream, and is oriented to the natural horizontal aspect ratio of the oropharyngeal region of the mouth. In one embodiment of the invention, the outline of the opening (135) resembles a bean.

The dry particle inhaler described herein may be used with medicament particles of low, medium, and high shear forces.

The dry particle inhaler and capsules described herein may be made with a variety of suitable materials known to those skilled in the art, such as metal, glass, rubber, and plastic.

While the invention has been described with reference to particular embodiments, those skilled in the art will be able to make various modifications without departing from the scope thereof.

For the avoidance of doubt, the disclosure hereof includes, in addition to the foregoing features, the features defined by the following numbered paragraphs:
1. A dry powder inhaler comprising:
   (a) an intake section; mechanically connected to
   (b) a mixing section; mechanically connected to
   (c) a mouthpiece;
   wherein the intake section, and mouthpiece each have a longitudinal axis; and
   wherein air flows through a passage extending from the intake section through the mixing section through the mouthpiece and means for regulating the flow rate of the air are present between the intake section and the mouthpiece.
2. The dry powder inhaler of Paragraph 1 wherein the mechanical connection between the mouthpiece and the mixing section comprises a swivel joint which allows the longitudinal axis of the intake section to be parallel to the longitudinal axis of the mouthpiece.
3. The dry powder inhaler of Paragraph 1 further comprising a cover mechanically connected to the dry powder inhaler, wherein the cover shelters the mixing section or mouthpiece.
4. The dry powder inhaler of Paragraph 4 wherein the mixing section cover only opens when the angle defined by the longitudinal axis of the intake section and the longitudinal axis of the mouthpiece and the swivel joint vertex is a fixed number of degrees.
5. The dry powder inhaler of Paragraph 4, wherein the fixed number of degrees the angle must be for the cover to be opened is between approximately ninety degrees and one hundred and eighty degrees.
6. The dry powder inhaler of Paragraph 3 wherein the mixing or storage section cover is translucent and is a magnifying lens.
7. The dry powder inhaler of Paragraph 1 further comprising a storage section mechanically connected to the dry powder inhaler, wherein a cover mechanically connected to the storage section shelters the storage section, and the cover may assume one or more fixed open positions.
8. The dry powder inhaler of Paragraph 7 wherein the storage section further includes mechanical fasteners mechanically connected to the storage section to secure capsules within the storage section.
9. The dry powder inhaler of Paragraph 1 comprising a mouthpiece sized to extend mid-way into the oral cavity of a user, the mouthpiece including a tongue depressor.
10. The dry powder inhaler of Paragraph 1 wherein the intake section includes an inner channel and comprises:
   (a) an intake port; covered by
   (b) a flow regulator; and
   (c) a bleed orifice,
   wherein the intake port and the bleed orifice both admit air to the dry powder inhaler, and regulate the rate of admission of the air.
11. The dry powder inhaler of Paragraph 1 wherein the mouthpiece opening has an approximately 3:1 horizontal aspect ratio.
12. A dry powder inhaler comprising a flow regulator comprising:
   (α) a piston comprising a piston head connected to a piston rod; and
   (β) one or more springs connected to the piston and the inner walls of the intake chamber; wherein the piston rod is wider at the proximal portion connected to the piston head and narrower at the distal portion; and
   wherein the piston head covers the intake port; wherein the piston head moves away from the intake port to admit air to the intake port, and wherein movement of the piston head is modulated by the springs connecting the piston to the inner walls of the intake chamber.
13. A dry powder inhaler comprising a feedback module connected to the intake chamber, wherein the feedback module generates an electrical signal in response to the flow of air in the intake chamber.
14. The dry powder inhaler of Paragraph 13, wherein the feedback module generates an audio signal.
15. The dry powder in haler of Paragraph 13 wherein the strength of the signal from the feedback module may be varied by a user of the dry powder inhaler.
16. A dry powder inhaler comprising an inlet, mixing chamber and mouthpiece, wherein the mixing section is a chamber which comprises a holder for a capsule having at least one of top and bottom keying portions; and the holder is nested inside the chamber; wherein the holder mechanically grips the top and bottom keying portions of the capsule, and wherein the holder opens the capsule when the angle defined by the longitudinal axis of the intake section and the longitudinal axis of the mouthpiece and the swivel joint vertex is a fixed number of degrees, and closes the capsule when the angle defined by the longitudinal axis of the intake section and the longitudinal axis of the mouthpiece and the swivel joint vertex is a fixed number of degrees.
17. The dry powder inhaler of Paragraph 16, wherein the fixed number of degrees needed to open the capsule is between approximately ninety degrees and one hundred and eighty degrees, and the fixed number of degrees to close the capsule is between approximately ninety and zero degrees.
18. The dry powder inhaler of Paragraph 16, wherein the holder only admits one capsule with one type of keying portion.
19. A dry powder inhaler comprising an inlet, mixing chamber, and mouthpiece, wherein the mixing chamber comprises a Venturi chamber comprising at least a tangential inlet or outlet that is shaped to give air passing through it a cyclonic flow.
20. The dry powder inhaler of Paragraph 19 comprising a Venturi chamber, wherein the Venturi chamber is curved or includes protrusions or spiral shapes to keep the air velocity below the inflection speed limit.
21. A capsule for holding a medicament for use in a dry powder inhaler comprising at least one of a keying surface to align the capsule in the dry powder inhaler.
22. A capsule for holding medicament for use in a dry powder inhaler comprising an inlet and an outlet in alignment to direct flow through the capsule in a cyclonic pattern.
23. The capsule of Paragraph 22 comprising a first tube and a second tube, wherein:
   (a) the first tube has a long axis, having an inner and an outer surface radial to the long axis, wherein the tube is open at one end perpendicular to the long axis and closed at one end perpendicular to the long axis; and wherein the first tube has at least one protrusion on its outer surface; and
   (b) the second tube has a long axis, having an inner and an outer surface radial to the long axis, wherein the tube is open at one end perpendicular to the long axis and closed at one end perpendicular to the long axis and wherein the second tube has at least one protrusion on its inner surface; and wherein the outer circumference of the first tube is approximately equal to the inner circumference of the second tube, such that the open end of the first tube can slide snugly into the open end of the second tube; and wherein a protrusion on the outer surface of the first tube may slide past a protrusion or slot on the inner surface of the second tube, locking the tubes together;
   wherein the first tube and the second tube each have one or more secondary holes other than the openings at the end of each tube, wherein at least one secondary hole in the first tube may be made coincident with at least one secondary hole in the second tube when the first tube is slid onto the second tube in the unlocked position by rotation of the first and second tubes about their long axes, and
   wherein when the first tube is locked onto the second tube at least two secondary holes in the first tube may be made coincident with at least two secondary holes in the second tube by rotation of the first and second tubes about their long axes.
24. The capsule of Paragraph 22 further comprising keying surfaces at the closed ends of the tubes.
25. The capsule of Paragraph 22 comprising structures in the interior surface of the capsule that create cyclonic air flow.
26. The capsule of Paragraph 25 wherein the structures are cone-shaped.
27. The capsule of Paragraph 22 comprising a slot to admit air flow to release medicament into the airstream of the dry particle inhaler wherein the vertical slot ratio of the hole is between 1:1 and 3:1.
28. The capsule of Paragraph 27, wherein the vertical slot ratio is 2:1.
29. The capsule of Paragraph 22 further including medicament selected from the group consisting of liquid, powder, and gaseous medicaments.
30. A medicament capsule for use in a dry powder inhaler comprising a fastening mechanism to attach it to a second medicament capsule.
31. The medicament capsule of Paragraph 30 wherein the fastening mechanism can attach the capsule to a storage compartment for capsules in an inhaler.
32. A method of making a capsule comprising a powdered medicament comprising the steps of:
   (a) introducing a liquid or gel containing medicament into a capsule comprising at least one inlet and an outlet in alignment to direct flow through the capsule in a cyclonic pattern, wherein the capsule comprises at least two pieces, which can be rotated relative to each other to close the inlet or outlet; and
   (b) drying the medicament to form a powder.
33. The dry particle inhaler of Paragraph 1, wherein the mixing section has a long axis, and wherein the air flowing through the mixing section to the mouthpiece exits the mixing section at a tangent to a circle described by a radius about the long axis of the mixing section.

## Claims

1. A medicament capsule (300) for an inhaler (10) wherein the medicament capsule (300) comprises holes (320;370;390) in the capsule to admit air flow, **characterise in that** the capsule comprises structures (375;445) in the interior surface of the capsule (300) which in use create cyclonic air flow.

2. A medicament capsule (300) according to Claim 1 wherein the structures are cone-shaped structures (375;445) or other cyclone-creating structures.

3. A medicament capsule according to Claim 1 wherein the said structures include a hole (320;370;390) to admit air flow to release medicament into the airstream of the dry particle inhaler **characterized in that** the vertical aspect ratio of the hole is between 1:1 and 3:1.

4. A medicament capsule (300) according to Claim 1 or Claim 3 at least one said hole (320;370;390) of which includes at least one bevelled edge.

5. The medicament capsule of Claim 3, wherein the vertical aspect ratio is 2:1.

6. The medicament capsule of Claim 4, wherein the holes are ellipse-shaped.

7. A capsule according to any preceding claim comprising first (310) and second (380) tubes that are retained one inside the other to define the capsule body, such that an end (330,400) of each said tube defines respectively a said top or bottom end of the capsule (300).

8. A capsule according to Claim 7 including a respective keying surface or fastening mechanism (360,430) on an end of one or both said tubes.

9. A capsule according to Claim 7 or Claim 8 wherein the first tube (310) and the second tube (380) each have an opening at an end (320,390); and one or more secondary holes (370,420) other than the openings at the end of each tube, wherein at least one secondary hole (370) in the first tube (310) may be made coincident with at least one secondary hole in the second tube (380), when the first tube (310) is inside the second tube (380), by rotation of the first and second tubes one inside the other.

10. A capsule according to Claim 9 wherein at least two secondary holes (370) in the first tube (310) may be made coincident with at least two secondary holes in the second tube (380) by rotation of the first and second tubes one inside the other.

11. A capsule according to any of Claims 7 to 10 wherein the first tube (310) has a long axis (340), and an inner and an outer surface radial to the long axis, wherein the tube (310) is open at one end (320) perpendicular to the long axis (340) and closed at one end (330) perpendicular to the long axis; and wherein the first tube (310) has at least one protrusion (350) on its outer surface; and
the second tube (380) has a long axis (410), and an inner and an outer surface radial to the long axis (410), wherein the tube is open at one end (390) perpendicular to the long axis (410) and closed at one end (400) perpendicular to the long axis (410) and wherein the second tube (380) has at least one protrusion on its inner surface; and wherein the outer circumference of the first tube (310) is approximately equal to the inner circumference of the second tube (380) such that the open end (320) of the first tube (310) can slide snugly into the open end (390) of the second tube (380); and wherein a protrusion (350) on the outer surface of the first tube (310) may slide past a protrusion on the inner surface of the second tube (380), locking the tubes together.

12. A medicament capsule (300) according to Claim 1 for holding medicament for use in a dry powder inhaler, the capsule comprising a generally cylindrical, hollow tube having top and bottom ends one of which is closed, the closed end including a keying surface or fastening mechanism that is mateable with a further component so as to permit engagement of the capsule with the further component, or identification of the capsule.

13. A capsule according to Claim 12 wherein the capsule includes a said keying surface at each of its top and bottom ends.

14. A capsule according to Claim 12 or Claim 13 wherein one or more keying surface or fastening mechanism is mateable with a keying surface of a dry particle inhaler.

15. A capsule according to any preceding claim further including medicament selected from the group consisting of liquid, powder, and gaseous medicaments.

16. A capsule according to Claim 15 wherein the medicament is a dry powder.

17. A capsule according to Claim 12 wherein the keying surface is mateable with a complementary keying surface or a second medicament capsule.

18. The capsule according to Claim 12 wherein the keying surface can attach the capsule to a storage compartment for capsules in an inhaler.

19. A capsule according to any preceding claim when inserted into the mixing section of a dry powder inhaler defining a Venturi adjacent the exterior of the capsule.

20. A capsule according to Claim 12 the shape of which co-operates with a closeable chamber of a dry powder inhaler in which the capsule is insertable such that closing of the chamber is possible only when the keying surface is aligned according to a chosen orientation.

21. A capsule according to Claim 12 wherein the keying surface provides an indication of a medicament contained within the capsule.

22. A method of making a capsule comprising a medicament comprising the step of introducing one or more chemicals into the capsule of Claim 1.

23. A method of making a capsule comprising a powdered medicament comprising the steps of:
(a) introducing a liquid or gel containing medicament into a capsule according to Claim 1; and
(b) drying the medicament to form a powder.

## Patentansprüche

1. Medikamentenkapsel (300) für ein Inhalationsgerät (10), wobei die Medikamentenkapsel (300) Löcher (320;370;390) in der Kapsel aufweist, um einen Luftstrom einzulassen, **dadurch gekennzeichnet, dass** die Kapsel Strukturen (375;445) in der Innenfläche der Kapsel (300) aufweist, die im Einsatz einen Luftwirbelstrom erzeugt.

2. Medikamentenkapsel (300) nach Anspruch 1, wobei die Strukturen konusförmige Strukturen (375;445) oder andere, Wirbelstrom-erzeugende Strukturen sind.

3. Medikamentenkapsel nach Anspruch 1, wobei die Strukturen ein Loch (320;370;390) aufweisen, um einen Luftstrom einzulassen, um ein Medikament in den Luftstrom des Trockenpartikel-Inhalationsgeräts abzugeben, **dadurch gekennzeichnet, dass** das vertikale Seitenverhältnis des Lochs zwischen 1:1 und 3:1 liegt.

4. Medikamentenkapsel (300)nach Anspruch 1 oder Anspruch 3, wobei mindestens ein Loch (320;370;390) derselben mindestens eine abgeschrägte Kante aufweist.

5. Medikamentenkapsel nach Anspruch 3, wobei das vertikale Seitenverhältnis 2:1 beträgt.

6. Medikamentenkapsel nach Anspruch 4, wobei die Löcher ellipsenförmig sind.

7. Kapsel nach einem der vorangehenden Ansprüche, mit ersten (310) und zweiten (380) Röhren, die ineinander gehalten sind, um den Kapselkörper festzulegen, so dass ein Ende (330,400) jeder Röhre jeweils ein oberes oder unteres Ende der Kapsel (300) festlegt.

8. Kapsel nach Anspruch 7 mit einer jeweiligen Verkeilungsfläche oder einem Befestigungsmechanismus (360,430) an einem Ende eines oder beider Röhren.

9. Kapsel nach Anspruch 7 oder Anspruch 8, wobei die erste Röhre (310) und die zweite Röhre (380) jeweils eine Öffnung an einem Ende (320,390) und ein oder mehrere Sekundärloch/Sekundärlöcher (370,420) außer den Öffnungen am Ende jeder Röhre aufweisen, wobei mindestens ein Sekundärloch (370) in der ersten Röhre (310) mit mindestens einem Sekundärloch in der zweiten Röhre (380), wenn sich die erste Röhre (310) innerhalb der zweiten Röhre (380) befindet, durch Drehen einer der ersten und zweiten Röhren innerhalb der anderen zum Koinzidieren gebracht werden kann.

10. Kapsel nach Anspruch 9, wobei mindestens zwei Sekundärlöcher (370) in der ersten Röhre (310) mit mindestens zwei Sekundärlöchern in der zweiten Röhre (380) durch Drehen einer der ersten und zweiten Röhren innerhalb der anderen zum Koinzidieren gebracht werden kann.

11. Kapsel nach einem der Ansprüche 7 bis 10, wobei die erste Röhre (310) eine Langachse (340) und eine innere und äußere Oberfläche radial zu der Langachse aufweist, wobei die Röhre (310) an einem zu der Langachse (340) senkrechten Ende (320) offen ist und an einem zu der Langachse senkrechten Ende (330) geschlossen ist, und wobei die erste Röhre (310) mindestens einen Vorsprung (350) an ihrer Außenfläche aufweist, und
die zweite Röhre (380) eine Langachse (410) sowie eine Innen- und Außenfläche radial zu der Langachse (410) aufweist, wobei die Röhre an einem zu der Langachse (410) senkrechten Ende (390) offen ist und an einem zu der Langachse (410) senkrechten Ende (400) geschlossen ist, und wobei die zweite Röhre (380) mindestens einen Vorsprung an ihrer Innenfläche aufweist, wobei der Außenumfang der ersten Röhre (310) in etwa gleich dem Innenumfang der zweiten Röhre (380) ist, so dass das offene Ende (320) der ersten Röhre (310) eng in das offene Ende (390) der zweiten Röhre (380) gleiten kann, und wobei ein Vorsprung (350) an der Außenfläche der ersten Röhre (310) an einem Vorsprung an der Innenfläche der zweiten Röhre (380) vorbeigleiten kann, wodurch.die Röhren miteinander verriegelt werden.

12. Medikamentenkapsel (300) nach Anspruch 1 zum Halten eines Medikaments zur Verwendung in einem Trockenpulver-Inhalationsgerät, wobei die Kapsel eine allgemein zylindrische, hohle Röhre mit oberen und unteren Enden aufweist, von denen eines geschlossen ist, und das geschlossene Ende eine Verkeilungsfläche oder einen Befestigungsmechanismus hat, der mit einer weiteren Komponente zusammensetzbar ist, so dass ein Eingriff der Kapsel mit der weiteren Komponente oder eine Identifizierung der Kapsel möglich ist.

13. Kapsel nach Anspruch 12, wobei die Kapsel die Verkeilungsfläche an jedem ihrer oberen und unteren Enden aufweist.

14. Kapsel nach Anspruch 12 oder Anspruch 13, wobei eine oder mehrere Verkeilungsfläche(n) oder Befestigungsmechanismen mit einer Verkeilungsfläche eines Trockenpartikel-Inhalationsgeräts zusammensetzbar ist.

15. Kapsel nach einem der vorangehenden Ansprüche, ferner mit einem Medikament, das aus der aus flüssigem, pulverförmigen und gasförmigen Medikamenten bestehenden Gruppe ausgewählt ist.

16. Kapsel nach Anspruch 15, wobei das Medikament ein Trockenpulver ist.

17. Kapsel nach Anspruch 12, wobei die Verkeilungsfläche mit einer komplementären Verkeilungsfläche oder einer zweiten Medikamentenkapsel zusammensetzbar ist.

18. Kapsel nach Anspruch 12, wobei die Verkeilungsfläche die Kapsel an einem Speicherfach für Kapseln in einem Inhalationsgerät anbringen kann.

19. Kapsel nach einem der vorangehenden Ansprüche, wenn sie in den Mischabschnitt eines Trockenpulver-Inhalationsgeräts eingesetzt ist, der einen Venturi angrenzend an die Außenseite der Kapsel festlegt.

20. Kapsel nach Anspruch 12, deren Form mit einer verschließbaren Kammer eines Trockenpulver-Inhalationsgeräts zusammenwirkt, in die die Kapsel so einsetzbar ist, dass das Schließen der Kammer nur dann möglich ist, wenn die Verkeilungsfläche entsprechend einer gewählten Ausrichtung ausgerichtet ist.

21. Kapsel nach Anspruch 12, wobei die Verkeilungsfläche eine Anzeige eines in der Kapsel enthaltenen Medikaments vorsieht.

22. Verfahren zum Herstellen einer Kapsel mit einem Medikament, umfassend den Schritt des Einführens einer oder mehrere Chemikalien in die Kapsel nach Anspruch 1.

23. Verfahren zum Herstellen einer Kapsel mit einem pulverförmigen Medikament, mit den folgenden Schritten:
(a) Einbringen einer/eines ein Medikament enthaltenden Flüssigkeit oder Gels in eine Kapsel nach Anspruch 1, und
(b) Trocknen des Medikaments, um ein Pulver zu bilden.

## Revendications

1. Capsule de médicament (300) pour un inhalateur (10) dans laquelle la capsule de médicament (300) comprend des trous (320 ; 370 ; 390) dans la capsule pour admettre un flux d'air, **caractérisée en ce que** la capsule comprend des structures (375 ; 445) dans la surface intérieure de la capsule (300) qui durant l'utilisation créent un flux d'air cyclonique.

2. Capsule de médicament (300) selon la revendication 1, dans laquelle les structures sont des structures en forme de cône (375 ; 445) ou autres structures de formation de cyclone.

3. Capsule de médicament selon la revendication 1, dans laquelle lesdites structures comprennent un trou (320 ; 370 ; 390) pour admettre un flux d'air pour libérer le médicament dans le courant d'air de l'inhalateur à particules sèches, **caractérisée en ce que** le rapport d'aspect vertical du trou est compris entre 1/1 et 3/1.

4. Capsule de médicament (300) selon la revendication 1 ou la revendication 3, dont au moins un desdits trous (320 ; 370 ; 390) comprend au moins un bord biseauté.

5. Capsule de médicament selon la revendication 3, dans laquelle le rapport d'aspect vertical est de 2/1.

6. Capsule de médicament selon la revendication 4, dans laquelle les trous sont en forme d'ellipse.

7. Capsule selon l'une quelconque des revendications précédentes, comprenant des premier (310) et second (380) tubes qui sont retenus l'un à l'intérieur de l'autre pour définir le corps de la capsule, de telle manière qu'une extrémité (330, 400) de chacun desdits tubes définit respectivement une dite extrémité supérieure ou inférieure de la capsule (300).

8. Capsule selon la revendication 7, comprenant une surface de manipulation respective ou un mécanisme de fixation respectif (360, 430) sur une extrémité de l'un desdits tubes ou des deux.

9. Capsule selon la revendication 7 ou la revendication 8, dans laquelle le premier tube (310) et le second tube (380) ont chacun une ouverture au niveau d'une extrémité (320, 390) ; et un ou plusieurs trous secondaires (370, 420) autres que les ouvertures au niveau de l'extrémité de chaque tube, dans laquelle il est possible de faire coïncider au moins un trou secondaire (370) dans le premier tube (310) avec au moins un trou secondaire dans le second tube (380), lorsque le premier tube (310) se trouve à l'intérieur du second tube (380), par rotation des premier et second tubes l'un à l'intérieur de l'autre.

10. Capsule selon la revendication 9, dans laquelle il est possible de faire coïncider au moins deux trous secondaires (370) dans le premier tube (310) avec au moins deux trous secondaires dans le second tube (380) par rotation des premier et second tubes l'un à l'intérieur de l'autre.

11. Capsule selon l'une quelconque des revendications 7 à 10, dans laquelle le premier tube (310) a un axe long (340), et une surface intérieure et une surface extérieure radiales par rapport à l'axe long, dans lequel le tube (310) est ouvert à une extrémité (320) perpendiculaire à l'axe long (340) et fermé à une extrémité (330) perpendiculaire à l'axe long ; et dans lequel le premier tube (310) a au moins une saillie (350) sur sa surface extérieure ; et
le second tube (380) a un axe long (410), et une surface intérieure et une surface extérieure radiales par rapport à l'axe long (410), dans laquelle le tube est ouvert à une extrémité (390) perpendiculaire à l'axe long (410) et fermé à une extrémité (400) perpendiculaire à l'axe long (410) et dans laquelle le second tube (380) a au moins une saillie sur sa surface intérieure ; et dans laquelle la circonférence extérieure du premier tube (310) est approximativement égale à la circonférence intérieure du second tube (380) de telle manière que l'extrémité ouverte (320) du premier tube (310) peut coulisser de manière serrée dans l'extrémité ouverte (390) du second tube (380) ; et dans laquelle une saillie (350) sur la surface extérieure du premier tube (310) peut coulisser devant une saillie sur la surface intérieure du second tube (380), ce qui verrouille les tubes l'un avec l'autre.

12. Capsule de médicament (300) selon la revendication 1, destinée à contenir un médicament destiné à être utilisé dans un inhalateur à poudre sèche, la capsule comprenant un tube creux généralement cylindrique ayant des extrémités supérieure et inférieure dont une est fermée, l'extrémité fermée comprenant une surface de manipulation ou un mécanisme de fixation qui peut s'apparier avec un autre composant de manière à permettre l'engagement de la capsule avec le composant supplémentaire, ou l'identification de la capsule.

13. Capsule selon la revendication 12, dans laquelle la capsule comprend une dite surface de manipulation au niveau de chacune de ses extrémités supérieure et inférieure.

14. Capsule selon la revendication 12 ou la revendication 13, dans laquelle un(e) ou plusieurs surfaces de manipulation ou mécanismes de fixation peut être apparié(e) avec une surface de manipulation d'un inhalateur à particules sèches.

15. Capsule selon l'une quelconque des revendications précédentes, comprenant en outre un médicament choisi dans le groupe constitué par des médicaments liquides, en poudre et gazeux.

16. Capsule selon la revendication 15, dans laquelle le médicament est une poudre sèche.

17. Capsule selon la revendication 12, dans laquelle la surface de manipulation peut être appariée avec une surface de manipulation supplémentaire ou une seconde capsule de médicament.

18. Capsule selon la revendication 12, dans laquelle la surface de manipulation peut fixer la capsule à un compartiment de stockage de capsules dans un inhalateur.

19. Capsule selon l'une quelconque des revendications précédentes lorsqu'elle est insérée dans la partie de mélange d'un inhalateur à poudre sèche définissant un diffuseur adjacent à l'extérieur de la capsule.

20. Capsule selon la revendication 12, dont la forme coopère avec une chambre pouvant être fermée d'un inhalateur à poudre sèche dans lequel la capsule peut être insérée de telle manière que la fermeture de la chambre est possible uniquement lorsque la surface de manipulation est alignée selon une orientation choisie.

21. Capsule selon la revendication 12, dans laquelle la surface de manipulation fournit une indication d'un médicament contenu à l'intérieur de la capsule.

22. Procédé de fabrication d'une capsule comprenant un médicament comprenant l'étape consistant à introduire un ou plusieurs produits chimiques dans la capsule selon la revendication 1.

23. Procédé de fabrication d'une capsule comprenant un médicament en poudre comprenant les étapes consistant à :
(a) introduire un liquide ou un gel contenant un médicament dans une capsule selon la revendication 1 ; et
(b) sécher le médicament pour former une poudre.
